# EUROPEAN PATENT APPLICATION

(11) **EP 2 320 342 A1**
(43) Date of publication of application: **11.05.2011**
(21) Application number: 10012341.3
(22) Date of filing: 25.07.2002
(51) Int. Cl.: G06F 19/00

(54) **System and method for creating data links between diagnostic and prescription information records**

(30) Priority: 08.08.2001 US 310794 P
(62) Divisional of application: 02756695.9
(71) Applicant: IMS Software Services, Ltd., Plymouth Meeting, PA 19462-0905 (US)
(72) Inventor: Ziegele, Stefan, 60528 Frankfurt am Main (DE); Howe, Marion, 60528 Frankfurt am Main (DE); Unger, Gerald, 05641-021 Sao paulo (BR); Banks, Timothy, Singapore 079119 (SG)
(74) Representative: Cohausz & Florack

(57) **Abstract**

A method and system for creating data links (130) between one or more diagnostic information records (150) and one or more prescription records (150) is disclosed. Initially, a relationship (110) between each of the diagnostic information records (150) and one or more, if any, of the prescription information records (150) is derived. Then, a correspondence probability (120) between one or more of the diagnostic information records (150) and one or more of the prescription information records (150) is determined using one or more derived relationships (110). Each of the diagnostic information records (150) is then linked to one or more of the prescription information records (150) using one or more correspondence probabilities (120).

## Description

### CROSS-REFERENCE TO RELATED APPLICATIONS

This application is based on Provisional Application Serial No. 60/310,794, filed August 8, 2001, which is incorporated herein by reference for all purposes and from which priority is claimed.

### BACKGROUND OF THE INVENTION

The present invention relates to medical software applications, and more particularly, to techniques for creating data links between different types of medical information records.

In the past, doctors used "pad-books" for recording patient visits. Pad-books are special forms in which doctors entered various data including a patient's name, age, sex and insurance carrier's information. The pad-books also contained records relating to both the patients' diagnostic information and prescription information corresponding to each diagnosis. When more than one diagnosis was made during a patient's visit, the doctor entered one or more prescriptions for each diagnosis in the pad-book.

In recent years, computers have become an integral part of hospitals and doctors' offices. A majority of doctors now keep most patient records in computers. Various data relating to patients' visits, including diagnostic information and prescription information, are entered and stored in the computer systems for easy retrieval.

When a new patient is examined by a doctor, certain patient-specific data, including the patient's name, address, sex, age, insurance carrier, and medical history are entered in a computer system and stored in a database. Upon completion of an examination, one or more diagnoses may be made for which medication is prescribed. Patient-specific diagnostic information and patient-specific prescription information are therefor entered into corresponding records in the doctor's computer system. Diagnostic information records and prescription information records are usually stored separately, and are updated whenever the patient visits the doctor.

Since diagnostic information records and prescription information records are kept in different databases, it is often hard to establish a clear link between diagnostic information and corresponding prescription information. Doctors rarely indicate such links in the patient computer records, and other staff may lack medical knowledge to properly determined correspondence between the prescribed medication and diagnoses. Moreover, when a patient with a chronic disease re-visits the doctor, the patient-visit records often do not contain any indication about the previously determined diagnosis for which a prescription is sought, as this is usually stored in a separate "patient history file" containing all previously determined diagnoses for the same patient.

Currently available medical software applications do not provide links between diagnostic information records and prescription information records. In recognition of the problem, a methodology which considers both diagnostic information records and prescription information records has been considered.

That methodology involves assigning prescribed products to diagnoses based on therapeutic indications derived from medical history data. Products having the same or similar indications are usually grouped in "therapeutic classes." For each therapeutic class, only a limited number of diagnoses are relevant. Similarly, for each diagnosis, only a limited number of therapeutic classes are of relevance.

Unfortunately, this methodology suffers from a drawback that it has a lower degree of accuracy. The products have heterogeneous therapeutic attributes, which result in similar but not necessarily equal therapeutic effects. Different products from the same therapeutic class can easily be used for different purposes. For example, two products, "Diane" (from Schering, AG, Berlin) and "Skid" (from Lichtenstein GmbH and Co., Mühlheim-Kärlich) belong to the same therapeutic class "D10B: Oral and anti-acne preparations." However, "Skid" is exclusively used for treating acne, and "Diane" is used predominantly as an "oral contraceptive," but it may also be used to treat acne. As a consequence, leading diagnoses per therapeutic class are not necessarily valid for each product in the entire class.

Furthermore, information is not updated by new data deliveries in this methodology. The "therapeutic class" approach works with patterns derived from historical data. The derived patterns are then applied on the actual data sample. If, in the actual data sample, there is a combination of the diagnostic and prescription information that does not exist in the historical data, then that combination must be manually linked. Therefore, the method does not have the ability to automatically link new combinations. For that reason, this methodology also cannot be used for new launches of medical services. Accordingly, there remains a need for a technique for creating accurate and automatically updated data linkage between diagnostic information records and prescription information records.

### SUMMARY OF THE INVENTION

An object of the present invention is to provide an accurate automated data linkage technique for linking diagnostic information records and prescription information records.

Another object of the present invention is to provide a data linkage technique which can be automatically updated.

In order to meet these and other objects of the present invention which will become apparent with reference to further disclosure set forth below, the present invention discloses a technique for creating data linkage between diagnostic information records and one or more prescription information records.

In one embodiment, a method for creating data links between a plurality of diagnostic information records and a plurality of prescription information records includes the steps of (a) analysing the plurality of diagnostic information records and the plurality of prescription information records to derive one or more diagnosis-to-prescription relationships, each relating to a group of one or more of the diagnostic information records to a group of the one or more prescription information records, if any; (b) determining one or more correspondence probabilities, each using a relationship derived in step (a) and indicating a correspondence between the group of one or more of the diagnostic information records and the group of one or more prescription information records; and (c) linking one or more of the diagnostic information records to one or more of the prescription information records using the one or more correspondence probabilities.

In a preferred embodiment, the method further includes the step of providing one or more historical relationships, where each of the historical relationships signifies a relationship between the group of diagnostic information records and the group of prescription information records, and where the determining step includes determining one or more correspondence probabilities, each using the one or more diagnosis-to-prescription relationships derived in step (a) and the one or more historical relationships.

In a highly preferred embodiment, a probability table is produced using the one or more correspondence probabilities for all diagnosis-prescription combinations of one or more diagnostic information records and one or more prescription information records.

In another embodiment of the present invention, a linking algorithm is applied to link diagnostic information records and prescription information records. The linking algorithm is preferably either a maximum-likelihood algorithm, or a relative-likelihood algorithm.

Finally, another advantageous aspect of the present invention provides for the step of automatically updating the correspondence probability.

### BRIEF DESCRIPTION OF THE DRAWINGS

Figure 1 is a flow diagram illustrating an exemplary method in accordance with the present invention.

Figure 2 is a flow diagram illustrating an exemplary method for generating and updating a probability table.

Figure 3 is a flow diagram illustrating a highly preferred methodology for implementing the linking step 130 of Fig. 1.

Figure 4 is a block diagram illustrating an exemplary system according to the present invention,

### DETAILED DESCRIPTION OF THE INVENTION

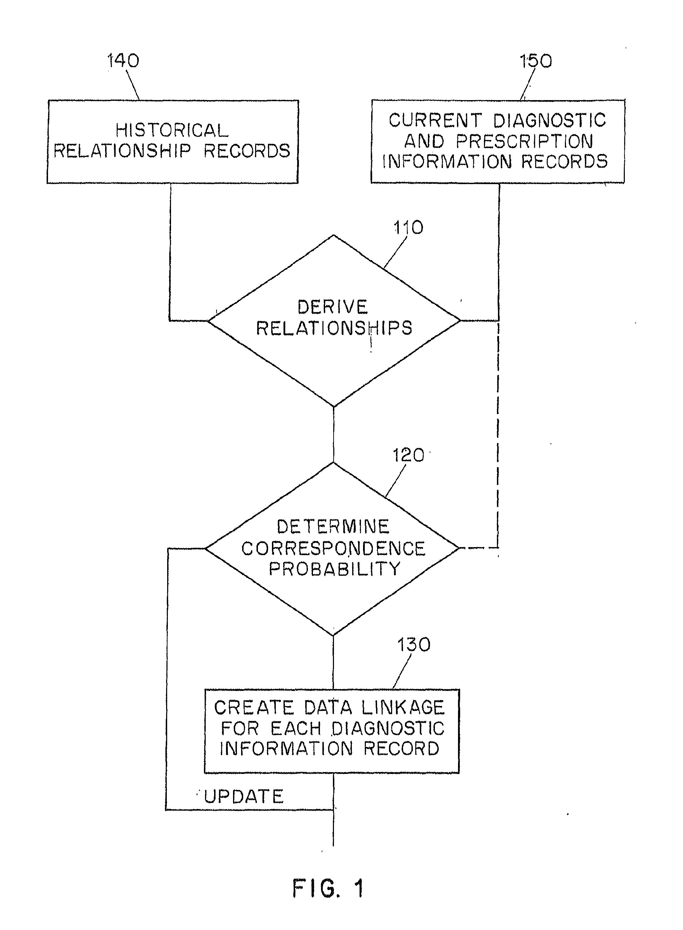

Figure 1 is a flow diagram illustrating an exemplary method 100 for creating data links between one or more diagnostic information records and one or more prescription information records. The method begins with deriving a relationship 110 between each diagnostic information record and one or more of the prescription information records. Next, a correspondence probability 120 between one or more of the diagnostic information records and one or more of the prescription information records is determined using one or more relationships derived in step 110. Subsequently, each of the diagnostic information records is linked 130 to one or more prescription information records using one or more correspondence probabilities determined in step 120.

The methodology requires the existence of diagnostic information records and prescription information records. In accordance with a preferred embodiment, a set of one or more historical relationship records are provided in step 140 prior to step 110.

Each historical relationship record signifies a relationship between one or more historical diagnostic information records and one or more historical prescription information records. Historical diagnostic information records and historical prescription information records represent, for example, diagnostic information determined by a pre-determined set of sample medical professionals within a pre-determined period of time. Historical prescription information records signify the prescription information corresponding to the historical diagnostic information. In other words, the historical relationship records contain data provided by a pro-determined set of sample doctors, who manually linked each historical diagnostic information record with one or more historical prescription information records.

Another set of records which may be used in step 110 are current diagnostic and prescription information records 150. The current diagnostic information records contain diagnostic information determined by doctors during patient visits. The current prescription information records contain the corresponding prescription information that resulted from such diagnosis.

Each diagnostic record may be related to one or more prescription records, A relationship in which one diagnostic information record is related to only one prescription information record is referred to as a one-to-one relationship. A relationship in which one diagnostic information record is related to more than one prescription information record is referred to as a one-to many relationship.

It is also possible that more than one diagnostic information record is related to only one prescription information record. This relationship is referred to as a many-to-one relationship. Similarly, a relationship in which more than one diagnostic information record is related to more than one prescription information record, is referred to as a many-to-many relationship.

In step 110, these relationships are determined, so that relevant current records can be identified. At the conclusion of step 110, a sub-set of current diagnostic and prescription information records 150 can be formed, containing only those diagnostic and prescription information records which are related to each other via either of a one-to-one and one-to-many relationships, as determined in step 110. This is accomplished by using an exemplary software procedure disclosed in the Appendix A.

In a preferred embodiment, both the historical relationship records 140 and the current diagnostic and prescription information records 150 may be used in step 110. These two sets of records are merged by utilizing an exemplary software program disclosed in the Appendix B,

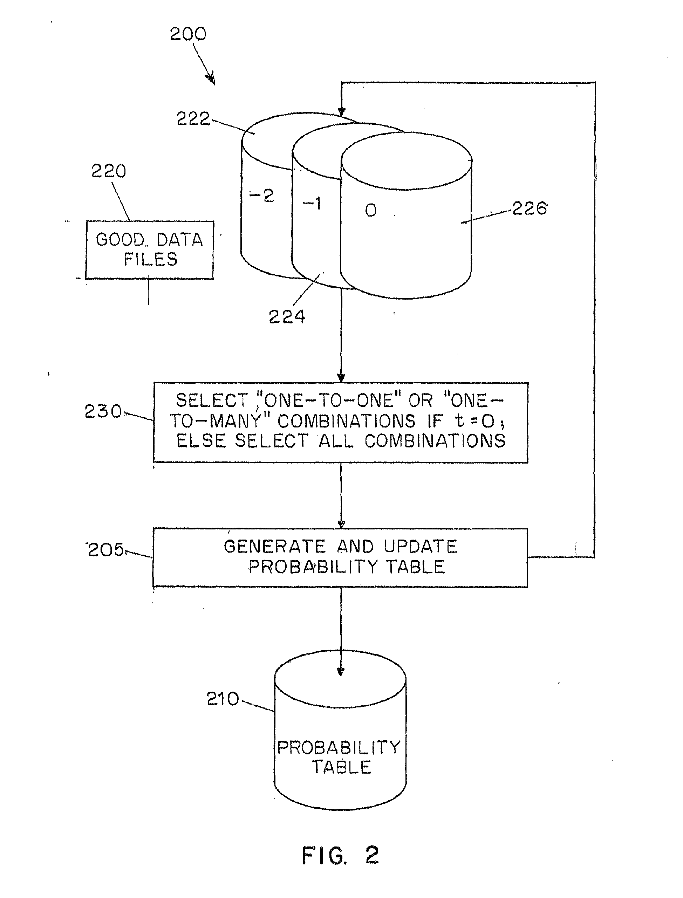

In a preferred embodiment, step 120 is achieved through the use of a probability table. Referring to Fig. 2, a flow diagram illustrating a method of generating and updating a probability table is provided, In the preferred embodiment, good data files 220 include both the historical relationship records 140 and the current diagnostic and prescription information records 150. The current diagnostic and prescription information records 150 are represented with Current Good Data File 226, for t=0. The exemplary historical information records are represented with Historical Good Data Files 222, and 224 for two preceding time periods, namely for t=-2 and t=-1, respectively. However, the good data files 220 may contain the historical information records 140 for any number of preceding time periods.

The probability table 210 is initially produced in step 205 by using the oldest good data file. In the exemplary set of good data files 220 from Fig. 2, the probability table is initially produced in step 205 by using the historical good data file 222. From the historical good data file 222, all combinations of diagnostic information records and prescription information records are determined. The good data file 222 may contain certain combinations of diagnostic and prescription information records that occur more frequently than others. Hence, a frequency of occurrence is also determined. The frequency of occurrence is determined from the historical good data file 222 by calculating how many times a particular diagnostic information record is combined with a particular prescription information record, and dividing that number with the total number of combinations in the historical good data file 222. Once all the combinations are determined, and the frequencies of occurrence are calculated, the probability table 210 is produced.

In addition to the frequency of occurrence, two ranks of occurrence are also determined for each determined combination of diagnostic and prescription information records. The first rank of occurrence is a diagnostic rank of occurrence R1. The diagnostic rank of occurrence R1 signifies a rank of occurrence of a particular diagnostic information record from a list of all diagnostic information records corresponding to a particular prescription information record. In other words, for each prescription information record there may be one or more diagnostic information records, which are ranked in descending order by their number of frequencies in the probability table, namely their diagnostic rank of occurrence R1. The highest diagnostic rank of occurrence is R2=1. In case that certain combinations of the particular diagnostic information record and the corresponding prescription information records have the same frequencies of occurrence, these combinations would also have the same rank R1. The diagnostic rank of occurrence R1 is preferably used for selecting the most likely combinations of diagnostic and prescription information records.

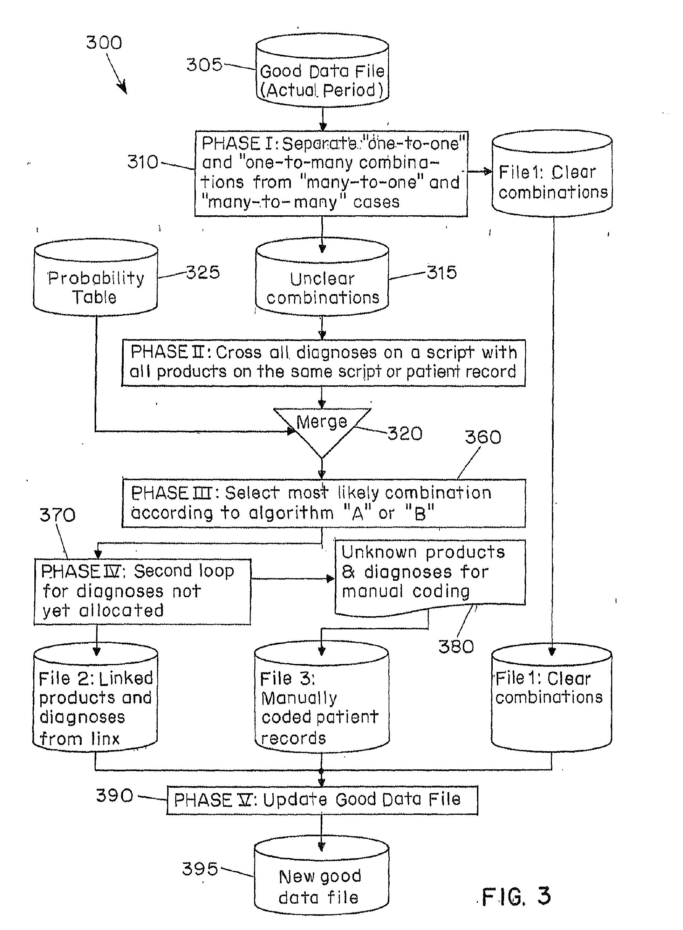

The second rank of occurrence is a prescription rank of occurrence R2. The rank of occurrence of a particular prescription information record R2 signifies a rank of occurrence of a particular prescription information record from a list of all prescription information records corresponding to a particular diagnostic information record. In other words, for each diagnostic information record there may be one or more prescription information records, which are ranked in descending order by their number of frequencies in the probability table, namely their prescription rank of occurrence R2. The highest prescription rank of occurrence is R2=1. In case that certain combinations of the particular prescription information record and the corresponding diagnostic information records have the same frequencies of occurrence, these combinations would also have the same rank R2. The prescription rank of occurrence is preferably used in a second loop algorithm (see Fig. 3, step 370). In the preferred embodiment, the assigning of the ranks of occurrence R1 and R2 is accomplished by using a "ProcRank" procedure, provided in an exemplary statistical analysis package "The SAS System", Version 6.090470P042699 for OS/390, manufactured by SAS Institute Inc., Cary, NC.

In a preferred embodiment, the probability table also includes a uniformly distributed random number for each determined combination of diagnostic and prescription information records. The uniformly distributed random number is used in case all combinations for a given case are equally ranked due to identical frequencies. For example, there may be, a case in which several combinations have frequencies of "1." In that case, all those combinations would have ranks R1 and R2 equal to "1," respectively. In that case, no decision can be made based on the ranks of occurrence for those combinations, so the uniformly distributed random numbers must be used. In the preferred embodiment, uniformly distributed random numbers are determined for each combination by using a RANUNI (0) procedure, which is also provided in the exemplary SAS statistical analysis package. This procedure assures that each determined random number between 0 and 1 has the same probability. Once the uniformly distributed random numbers are calculated for each combination of the diagnostic and prescription information records, the combinations may be ranked in, for example, descending or ascending order.

An exemplary format of the probability table is shown in Table A:

**TABLE A**

| **VARIABLE** | **DESCRIPTION** | **Type** | **FROM** | **TO** |
|---|---|---|---|---|
| PFC | IMS product code | NUM | 001 | 007 |
| DIAG | ICD10 code for diagnosis | CHAR | 009 | 013 |
| FREQ | Frequency of occurrence | NUM | 015 | 024 |
| CYC | Production cycle | NUM | 026 | 029 |
| RANKRX (R1) | Rank of different diagnoses by product | NUM | 031 | 034 |
| RANKDX (R2) | Rank of different products by diagnosis | NUM | 036 | 039 |
| RANDOM | Random number | NUM | 041 | 045 |

In the preferred embodiment, the step of initially producing the probability table 205 is performed by using a ProbGen procedure, an exemplary software program shown in Appendix B. ProbGen is written in SAS programming language and includes the following steps:
a. reading a good data file;
b. calculating a frequency of occurrence;
c. determining diagnostic rank of occurrence R1;
d. determining prescription rank of occurrence R2
e. generating a probability table;
   (i) determining random numbers in stalemate situations;
   (ii) assigning untreated diagnoses to lowest rank (exceptional rule);
      and
   (iii) creating cells:
      1. product;
      2. diagnosis;
      3. frequency;
      4. cycle of good data file;
      5. diagnostic rank of occurrence R1;
      6. prescription rank of occurrence R2;
      7. random number.
         It is important to note that the step of reading good data file may optionally allow for reading of only one-to-one and one-to-many combinations of diagnostic and prescription information records. Also, the step of calculating Frequency of occurrence may be performed by using a "Summary" procedure, provided in the exemplary SAS statistical analysis package. The step of determining the diagnostic rank of occurrence is performed by using a "RANK" procedure, where "product" is a grouping parameter. The RANK procedure is also provided in the exemplary SAS statistical analysis package manufactured by SAS Institute Inc., Cary, NC. Similarly, the step of determining the prescription rank of occurrence is performed by using a "RANK" procedure, where "diagnosis" is a grouping parameter.

Once the probability table 210 is produced in step 205 by using the oldest good data file, it is updated, also in step 205, using the more recent good data files. In the exemplary set of good data files 220 from Fig. 2, the probability table is subsequently updated by using the historical good data file 224 and the current good data file 226. The updating procedure is similar to that of producing the probability table, except that when the frequencies of occurrence are determined for the more recent good data file, they are merged with the frequencies of occurrence from the existing probability table and all frequencies are summed up for each determined combination of diagnostic and prescription information records. In case that new combinations of diagnostic and prescription information are determined, they are added in the probability table. From the sumned-up frequencies, new ranks of occurrence R1 and R2, and new uniformly distributed random numbers are determined.

In the preferred embodiment, the step of updating the probability table 205 is performed by using a PROBUPD procedure, a software program shown in Appendix C. PROBUPD is also written in SAS programming language, and includes the following steps:
a. reading a good data file;
b. calculating a frequency of occurrence;
c. reading a previous probability table;
d. merging the previous probability table with the good data file;
   (i) accumulating frequencies for each combination of diagnostic and prescription information records;
   (ii) inserting new combinations of diagnostic and prescription information records with their frequencies from the good data file;
e. determining updated diagnostic ranks of occurrence R1;
f. determining updated prescription ranks of occurrence R2;
g. updating the probability table;
   (i) determining random numbers in stalemate situations;
   (ii) assigning untreated diagnoses to lowest rank (exceptional rule); and
   (iii) creating cells:
      1. product;
      2. diagnosis;
      3. frequency;
      4. cycle of good data file;
      5. diagnostic rank of occurrence R1;
      6. prescription rank of occurrence R2;
      7. random number.
         It is important to note that the step of reading good data file may optionally allow for reading of only one-to-one and one-to-many combinations of diagnostic and prescription information records. Also, the step of calculating frequency of occurrence may be performed by using the before-mentioned "Summary" procedure, and the step of determining the diagnostic rank of occurrence is performed by using the "RANK" procedure, where "product" is a grouping parameter, and the step of determining the prescription rank of occurrence is performed by using the "RANK" procedure, where "diagnosis" is a grouping parameter.

The method 200 also has an optional step 230 for selecting combinations of diagnostic and prescription information records having one-to-one or one-to-many relationships. This step 230 is used with respect to current diagnostic and prescription information records 150 (Fig. 1). In the exemplary embodiment of Fig. 2, the step 230 is used with respect to the good data file 226, and filters out the combinations of diagnostic and prescription information records having many-to-one and many-to-many relationships. The remaining combinations are used to update the probability table 210. The same updating procedure described above is used with respect to the remaining combinations.

It is preferable to use at least three good data files 220 for generating and updating the probability table 210, as provided in the exemplary embodiment of Fig. 2. Using at least three good data files 220 allows for higher statistical confidence of the results. However, if the historical information cannot be obtained or does not exist, the probability table may be produced by using the combinations having one-to-one and one-to-many relationships obtained from one or more sampling rounds.

Fig. 3 is a flow diagram illustrating a highly preferred methodology for implementing the linking step 130 of Fig. 1. In this preferred embodiment, the linking step 300 includes the step 310 of separating all combinations of diagnostic and prescription information records, having one-to-one or one-to-many relationships in the good data file 305, from the remaining combinations 315. Following the separating step, in step 320, each of the remaining combinations 315 is mapped with a respective data record in the probability table 325. Following the mapping step, in step 360, a linking algorithm is applied for automatically linking each of said prescription information records with each of said diagnostic information records. Subsequent to automatic linking step, in step 380, all the remaining unlinked records are manually linked. Finally, all the links in the good data file 305 are updated 390, and saved in a new good data file 395.

As said before, in step 310, all combinations of diagnostic and prescription information records having one-to-one or one-to-many relationships from the good data file 305 are separated from the remaining combinations 315. All the combinations having one-to-one or one-to-many relationships have been integrated already in the probability table during the updating step 205 by using the previously-mentioned "PROBUPD" procedure. The remaining combinations of diagnostic and prescription information records having many-to-one and many-to-many relationships are mapped in step 320 with the respective data records in the probability table 325. For example, if there are two diagnoses (D₁ D₂) and three products (P₁ - P₃), the following combinations are produced:

| **Presc/Diag** | **D₁** | **D₂** |
|---|---|---|
| **P₁** | P₁ D₁ | P₁ D₂ |
| **P₂** | P₂ D₁ | P₂ D₂ |
| **P₃** | P₃ D₁ | P₃ D₂ |

For each combination Pᵢ Dⱼ, the respective data record from the probability table is selected, holding information on frequencies of occurrence, ranks R₁ and R₂ as well as a random number. If any combination is not found in the table, it is considered a nonvalid combination and, thus, it falls off the list of possible combinations.

Following the mapping step, in step 360, a linking algorithm is applied for automatically linking each of said prescription information records with one or more of said diagnostic information records. There are two linking algorithms that are preferably used to automatically link the diagnostic and prescription information records.

One of the linking algorithms is a maximum likelihood algorithm. The maximum likelihood algorithm works by selecting the combination with a maximum likelihood of occurrence (highest rank) for each prescription information record. The maximum likelihood algorithm always selects the highest rank of occurrence, independent of its position relative to the second highest one. This best approximates the decision process of human operators.

As previously indicated, a diagnostic rank of occurrence R₁ is assigned to each mapped combination. For any given prescription information record, the combination with the highest diagnostic rank of occurrence R1 is selected by this algorithm. If, for example, two combinations have the same diagnostic ranks of occurrence R1, the same algorithm is applied with respect to prescription ranks of occurrence R₂. In that case, the combination with the highest prescription rank of occurrence is selected. If, for example, those two combinations also have the same prescription ranks of occurrence R2, a uniformly distributed random number decides upon selection. For equally ranked combinations, the one with the lowest random number is selected in the preferred embodiment. Alternatively, the combination with the highest random number may also be chosen.

An exemplary maximum-likelihood algorithm is a LinxA algorithm, a software program shown in Appendix D. LinxA is written in SAS programming language, but it may be written in any other programming language. LinxA processes the combinations of prescription and diagnostic information records having many-to-one and many-to-many relationships from the good data file and assigns the prescription information records to the corresponding diagnostic information records according to their highest rank. LinxA has the following steps:
a. reading a good data file ("Data Step");
b. filtering the combinations having one-to-one and one-to-many relationships;
   (i) determining the combinations having one-to-one and one-to-many relationships ("Summary" procedure);
   (ii) storing such combinations to a separate data file not subject to further processing;
c. creating all possible combinations of the remaining diagnostic and prescription information records ("SQL" procedure);
d. reading updated probability table;
e. merging probability table with SQL-file holding all possible combinations of the remaining diagnostic and prescription information records;
f. choosing for each prescription information record the diagnostic information record with the highest rank R1 and creating links;
g. checking for "lost diagnoses" where ranks R1 are equal;
h. repeating steps c, e, f, g using rank R2;
i. using uniformly distributed random number to make final decisions;
   and
j. releasing for printing and manually assignment diagnostic information records which cannot be assigned.

While the maximum likelihood algorithm assures a highest selection consistency with the intuitive decisions made by human beings in the same decision process, there may be reasons to select other combinations of prescription information records and diagnostic information records, When using the maximum-likelihood algorithm, the combinations with the highest probabilities are always chosen. This may, sometimes, lead to an overestimation of certain combinations, whereas others may slowly disappear from the audit. If a determined combination varies from the real combination, this deviation is represented as "bias." A relative likelihood algorithm assures maximum heterogeneity of the results and it reduces the bias because second-best combinations of diagnostic and prescription information records have a certain (non-zero) chance to be selected.

The relative likelihood algorithm selects the combination according to its proportion to other combinations by means of uniformly distributed relative-likelihood random numbers. The relative likelihood algorithm ignores ranks of occurrence and uses accumulated frequencies instead. Based on the accumulated frequencies, accumulated probabilities (between 0 and 1) are calculated. For each prescription information record, a uniformly distributed relative-likelihood random number between 0 and 1 is generated which sets the selection point from the accumulated distribution across diagnostic information records. The uniformly distributed relative-likelihood random numbers are generated using the RUNUNI(0) procedure, a standard function provided in the SAS.

An exemplary relative-likelihood algorithm is a LinxB algorithm, a software program shown in Appendix E. LinxB is also written in SAS programming language, but it may be written in any other programming language. LinxB processes the combinations of prescription and diagnostic information records having many-to-one and many-to-many relationships from the good data file and assigns the prescription information records to the corresponding diagnostic information records according to the accumulated rank principle. LinxB has the following steps:
a. reading a good data file ("Data Step");
b. filtering the combinations having one-to-one and one-to-many relationships;
   (i) determining the combinations having one-to-one and one-to-many relationships ("Summary" procedure);
   (ii) storing such combinations to a separate data file not subject to further processing;
c. creating all possible combinations of the remaining diagnostic and prescription information records ("SQL procedure);
d. reading updated probability table;
e. merging probability table with SQL-file holding all possible combinations of the remaining diagnostic and prescription information records;
f. calculating a uniform distribution relative-likelihood random number for each prescription information record;
g. for each prescription information record, accumulating frequencies of combinations with respective diagnostic information records, and selecting a particular combination over others by using the corresponding uniform distribution relative-likelihood random numbers;
h. repeating steps c and e; and
i. selecting for each prescription information record a corresponding diagnostic information record having the highest rank R2 from the probability table and creating links.

The individual steps of the relative likelihood algorithm are illustrated by using the following example. Assume that there are 5 diagnostic information records and 5 prescription information records. This does not necessarily mean that each prescription needs to be assigned to exactly one diagnostic information record; there could be diagnostic information records which are not combined with any prescription information records, and others with which two or more prescription information records have been combined. The exemplary frequencies of occurrence from the probability table are provided in Table B, and the corresponding probabilities are illustrated in Table C:

**TABLE B**

| **PRESC./DIAG.** | **D₁** | **D₂** | **D₃** | **D₄** | **D₅** | **TOTAL** |
|---|---|---|---|---|---|---|
| **P₁** | 120 | 50 | 30 | 10 | 10 | 220 |
| **P₂** | 100 | 100 | 30 | 20 | 0 | 250 |
| **P₃** | 30 | 50 | 20 | 0 | 0 | 100 |
| **P₄** | 10 | 20 | 70 | 10 | 10 | 120 |
| **P₅** | 0 | 0 | 0 | 60 | 90 | 150 |
| **Total** | 260 | 220 | 150 | 100 | 110 | 840 |

**TABLE C**

| **PRESC./DIAG.** | **D₁** | **D₂** | **D₃** | **D₄** | **D₅** | **TOTAL** |
|---|---|---|---|---|---|---|
| **P₁** | 0.5455 | 0.2273 | 0.1364 | 0.0455 | 0.0455 | 1.0000 |
| **P₂** | 0.4000 | 0.4000 | 0.1200 | 0.0800 | 0.0000 | 1.0000 |
| **P₃** | 0.3000 | 0.5000 | 0.2000 | 0.0000 | 0.0000 | 1.0000 |
| **P₄** | 0.0833 | 0.1667 | 0.5833 | 0.0833 | 0.0833 | 1.0000 |
| **P₅** | 0.0000 | 0.0000 | 0.0000 | 0.4000 | 0.6000 | 1.0000 |
| **Total** | 0.3095 | 0.2619 | 0.1786 | .01190 | 0.1310 | 1.0000 |

In this example, the combination **P₁D₁** has the highest probability (54.55%), which means that 54.55% of all possible uniformly distributed relative likelihood random numbers between 0 and 1 would fall into an interval [0.0000; 0.5455]. The next combination is **P₁D₂** has a probability of 0.2273, which means that 22.73% of all uniformly distributed relative-likelihood random numbers between 0 and 1 would fall in the interval [0.5456; 0.7727], and so on. Therefore, in 22.73% of all random number generations, the combination **P₁D₂** is selected. If the maximum likelihood algorithm is used, **P₁D₁** would always be chosen, ignoring the fact that also the combination **P₁D₂** has a probability of occurrence in roughly 23% of all cases. According to the maximum likelihood algorithm, **P₁D₂** receives a probability of "0" if it is actually less probable than **P₁D₁.** The linking of diagnostic information records and prescription information records according to the maximum likelihood algorithm is illustrated in Table D:

**TABLE D**

| **PRESC./DIAG.** | **D₁** | **D₂** | **D₃** | **D₄** | **D₅** |
|---|---|---|---|---|---|
| **P₁** | X | | | | |
| **P₂** | X | X | | | |
| **P₃** | | X | | | |
| **P₄** | | | X | | |
| **P₅** | | | | | X |

The relative likelihood algorithm may produce the same or different results. As said before, it uses accumulated frequencies instead, determined from the prescription information record frequencies. Based on the accumulated frequencies, accumulated probabilities (between 0 and 1) are calculated. For each prescription information record, a uniformly distributed relative-likelihood random number between 0 and 1 is generated which helps in selecting the appropriate diagnostic information record. In this example, the resulting accumulated probabilities are illustrated in Table E:

**TABLE E**

| **PRESC./IDIAG.** | **D₁** | **D₂** | **D₃** | **D₄** | **D₅** |
|---|---|---|---|---|---|
| **P₁** | 0.5455 | 0.7727 | 0.9091 | 0.9545 | 1.0000 |
| **P₂** | 0.4000 | 0.8000 | 0.9200 | 1.0000 | 1.0000 |
| **P₃** | 0.3000 | 0.8000 | 1.0000 | 1.0000 | 1.0000 |
| **P₄** | 0.0833 | 0.2500 | 0.8333 | 0.9167 | 1.0000 |
| **P₅** | 0.0000 | 0.0000 | 0.0000 | 0.4000 | 1.0440 |

For prescription information record P₁, the diagnostic information record D₁ is chosen only if the externally determined uniform distribution relative-likelihood random number is less than 0.5455 (equivalent to "54.55% of all cases"). In the given example, it is greater, but less than 0.7727, so diagnosis D₂ is selected. The linking of diagnostic information records and prescription information records according to the relative likelihood algorithm is illustrated in Table F:

**TABLE F**

| **PRESC./DIAG.** | **RANDOM** | **D₁** | **D₂** | **D₃** | **D₄** | **D₅** |
|---|---|---|---|---|---|---|
| **P₁** | 0,5706 | | X | | | |
| **P₂** | 0,0486 | X | | | | |
| **P₃** | 0,4491 | | X | | | |
| **P₄** | 0,5731 | | | X | | |
| **P₅** | 0,8929 | | | | | X |

Since the random numbers are uniformly distributed, P₁D₂ still has a probability of 0.5455 for being selected (any random number between 0 and 0.5455) but it will not automatically receive 1.0000 if p(P₁ D₂) > p(P₁ D₂), where "p" stands for 'probability'. Therefore, each diagnosis has a probability of being selected in proportion to its relative frequency in relation to other diagnoses. Hence uniform distribution relative-likelihood random numbers bring in an element of randomness in the selection process, but always according to the probability function of the respective combinations of diagnostic and prescription information records.

From the example given above, the following combinations of products and diagnoses would result, depending on whether the maximum-likelihood or relative-likelihood algorithm is used:

**TABLE G**

| **Presc.t** | **Max. Likelihood** **ALGORITHM** | **Rel. Likelihood** **ALGORITHM** |
|---|---|---|
| **P₁** | D₁ | D₂ |
| **P₂** | D₁ or D₂ | D₁ |
| **P₃** | D₂ | D₂ |
| **P₄** | D₃ | D₃ |
| **P₅** | D₄ | D₅ |

Given the random numbers listed above, the only difference between the maximum- and relative-likelihood algorithms exists with respect to the prescription information record P₁. In that case, the maximum-likelihood algorithm selects D₁ whereas the relative-likelihood algorithm selects D₂. For P₂, the relative likelihood algorithm arrives at an unequivocal link with D₁, whereas the maximum likelihood algorithm requires a second loop algorithm (see Fig. 3, step 370 described below). In both cases, the diagnostic information record D₄ is left unassigned ("diagnosis without prescription"). Only if P₄ receives a uniform distribution relative-likelihood random number of ≤ 0.4, it is selected for D₄.

If in the selection process no decision can be made for one or the other combination of diagnostic and prescription information records, a second loop algorithm may be applied, using slightly different criteria for arriving at more certain decisions. The same is valid if two or more diagnostic information are similar. In this case, it is possible that all products get assigned to only one diagnostic information record, particularly when working with the maximum likelihood algorithm. This would possibly generate too high a number of "diagnoses without therapy".

In order to clarify when the second loop algorithm may be used, consider the following examples in which two prescription and two diagnostic information records are combined. In the first example, no second loop is required. The ranks of occurrence and random numbers are illustrated in Table H:

**TABLE H**

| DIAG | Product | R1 | R2 | Random |
|---|---|---|---|---|
| D₁ | P₁ | 02 | 12 | 0.590 |
| D₁ | P₂ | 25 | 22 | 0.510 |
| D₂ | P₁ | 05 | 03 | 0.037 |
| D₂ | P₂ | 20 | 01 | 0.094 |

For P₁, diagnostic information record D₁ is selected because R1(P₁D₁)<R1(P₁D₂) For P₂, diagnostic information record D₂ is selected because R1(P₂D₂)<R1(P₂D₁). No second loop is needed in this case because both product and diagnostic information records are clearly linked.

The second example illustrates when the second loop algorithm may be used. The ranks of occurrence and random numbers for the second example are illustrated in Table I:

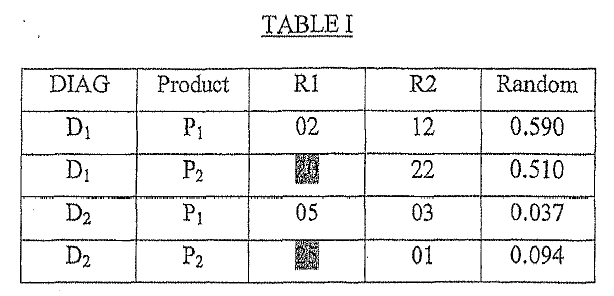

Both prescription information records P1 and P2 are assigned to a diagnostic information record D1, since R1(P1,D1)<R1(P1,D2) and R1(P2,D1)<R1(P2,D2). For D2, the ranks R2 of products prescribed for this diagnosis are checked. P1 is at position 3 and P2 at position 1, so P2 is re-linked to D2.

The third example illustrates that when the second loop does not provide data links, random number selection is used. The ranks of occurrence and random numbers for the third example are illustrated in Table J:

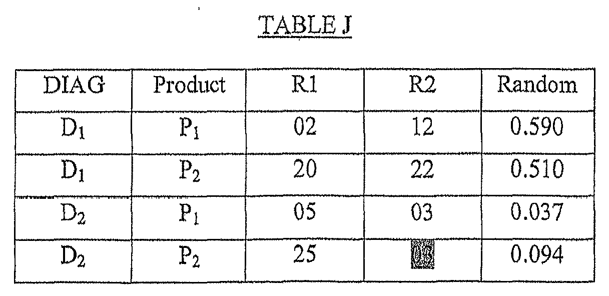

The diagnostic information record D2 could not be linked to any prescription information record in the first loop. In the second loop, the two prescription information records P1 and P2 turn out to have the same rank R2 (03) for D2. In this case, P1 will be selected for D2 because the random number in the probability table is lower (0.037 < 0.094).

As previously indicated, if in the selection process no decision can be made for one or the other combination of diagnostic and prescription information records, or if two or more diagnostic information are similar, a second loop step 370 may be applied. In step 370, the prescription rank of occurrence R₂ is used for linking the diagnostic and prescription information records. The prescription ranks of occurrence R2 are calculated based on the same frequencies from the probability table. However, the ranks are now arranged according to the prescription information records corresponding to a particular diagnostic information record (the summed frequency of all prescription information records relating to the same diagnostic information record equals 100%). If any of the prescription information records has a higher rank R2 with the *unassigned* diagnostic information record compared to the diagnostic information record to which it was linked in step 360, an overwrite is done and the new combination is selected for this specific prescription information record.

This can be illustrated by using the example of 5 prescription information records and 5 diagnostic information records illustrated before. As previously said, no product has been assigned to diagnosis D₄, neither by using the maximum likelihood, nor by using the relative likelihood algorithm. If all probabilities are determined by using the frequencies and diagnostic information (vertical), instead of prescription information (horizontal), a new set of probabilities and cumulative probabilities emerge. The exemplary probabilities are illustrated in Table K:

**TABLE K**

| **PROD./IDIAG.** | **D₁** | **D₂** | **D₃** | **D₄** | **D₅** | **TOTAL** |
|---|---|---|---|---|---|---|
| **P₁** | 0,4615 | 0,2273 | 0,2000 | 0,1000 | 0,0909 | 0,2619 |
| **P₂** | 0,3846 | 0,4545 | 0,2000 | 0,2000 | 0,0000 | 0,2976 |
| **P₃** | 0,1154 | 0,2273 | 0,1333 | 0,0000 | 0,0000 | 0,1190 |
| **P₄** | 0,0385 | 0,0909 | 0,4667 | 0,1000 | 0,0909 | 0,1429 |
| **P₅** | 0,0000 | 0,0000 | 0,0000 | 0,6000 | 0,8182 | 0,1786 |
| **Total** | 1,0000 | 1,0000 | 1,0000 | 1,0000 | 1,0000 | 1,0000 |

In this case, P₅ would be selected for D₄, however, since this would leave D₅ unassigned (the one with higher P₅ probabilities horizontally and vertically), D₄ will remain as the "diagnosis without prescription" (exceptional rule).

It is preferred that the second loop step 370 work according to the maximum likelihood algorithm, choosing the prescription information with the highest probability for the particular diagnostic information record, although the relative likelihood algorithm may also be used.

Subsequent to automatic linking step 360, in step 380, all the remaining unlinked records are manually linked. Such records may include a new prescription information record discovered in the application phase 300, a very rare or new diagnostic information record or a new indication for an existing prescription. Finally, all the links in the good data file 305 are updated 390, and saved in a new good data file 395.

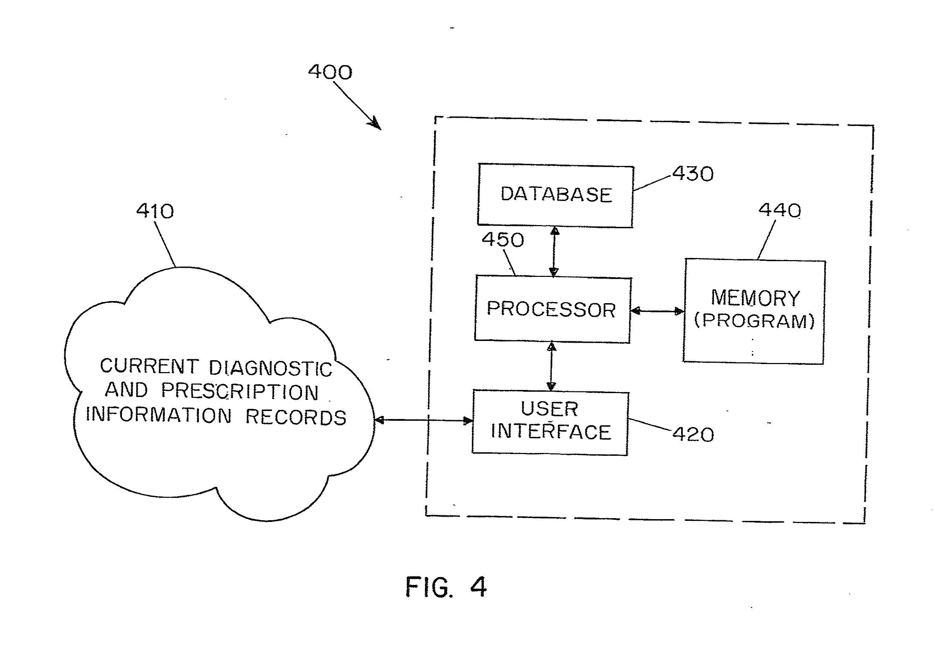

Referring to Fig. 4, a simplified block diagram of an exemplary system 400 according to the present invention is illustrated. The system 400 receives current diagnostic and prescription information records 410 via a user interface 420. The current diagnostic and prescription information records 410 may be stored in a database 430. In addition to current diagnostic and prescription information records 410, the database 430 may also store historical information records. A computer memory 440 contains one or more programs for deriving a relationship between one or more diagnostic information records and one or more prescription information records and for determining a correspondence probability between one or more diagnostic information records and one or more prescription information records using the derived relationships. The memory 440 also contains programs for linking each of the diagnostic information records with one or more prescription information records. A processor 450 is used to execute the programs stored in the memory 440. The resulting data may be stored in the database 430, and provided as an output via user interface 420.

The foregoing merely illustrates the principles of the invention. Various modifications and alterations to the described embodiments will be apparent to those skilled in the art in view of the teachings herein. It will thus be appreciated that those skilled in the art will be able to devise numerous techniques which, although not explicitly shown or described herein, embody the principles of the invention and are thus within the spirit and scope of the invention.
Embodiment 1 constitutes a method for creating data links between a plurality of diagnostic information records and a plurality of prescription information records, comprising the steps of: a) analyzing said plurality of diagnostic information records and said plurality of prescription information records to derive one or more diagnosis-to-prescription relationships, each relating a group of one or more of said diagnostic information records to a group of one or more, if any, of said prescription information records; b) determining one or more correspondence probabilities, each using a relationship derived in step (a) and indicating a correspondence between a group of one or more of said diagnostic information records and a group of one or more of said prescription information records; and c) linking one or more of said diagnostic information records to one or more of said prescription information records using said one or more correspondence probabilities determined in step (b).
Embodiment 2 constitutes a method, in particular of embodiment 1, further comprising the step of providing one or more historical relationships prior to step (a), each signifying a relationship between a group of one or more of said diagnostic information records and a group of one or more of said prescription information records, and wherein said step (b) further comprises determining one or more correspondence probabilities, each using one or more of said diagnosis-to-prescription relationships derived in step (a) and said one or more historical relationships.
Embodiment 3 constitutes a method , in particular of embodiment 1, wherein said diagnosis-to-prescription relationships comprise one-to-one relationships.
Embodiment 4 constitutes a method, in particular of embodiment 1, wherein said diagnosis-to-prescription relationships comprise one-to-many relationships.
Embodiment 5 constitutes a method, in particular of embodiment 1, wherein said diagnosis-to-prescription relationships comprise many-to-one relationships.
Embodiment 6 constitutes a method , in particular of embodiment 1, wherein said diagnosis-to-prescription relationships comprise many-to-many relationships.
Embodiment 7 constitutes a method, in particular of embodiment 1, wherein said step (b) includes producing a probability table using said one or more correspondence probabilities.
Embodiment 8 constitutes a method, in particular of embodiment 7, wherein said step of producing a probability table comprises determining one or more diagnosis-prescription combinations using said plurality of diagnostic information records and said plurality of prescription information records.
Embodiment 9 constitutes a method , in particular of embodiment 8, wherein said step of producing a probability table further comprises calculating a frequency of occurrence for each of said one or more diagnosis-prescription combinations.
Embodiment 10 constitutes a method , in particular of embodiment 9, wherein said step of producing a probability table further comprises using said frequency of occurrence to determine a diagnostic rank of occurrence for each of said one or more diagnosis-prescription combinations.
Embodiment 11 constitutes a method, in particular of embodiment 10, wherein said step of producing a probability table further comprises using said frequency of occurrence to determine a prescription rank of occurrence for each of said one or more diagnosis-prescription combinations.
Embodiment 12 constitutes a method, in particular of embodiment 11, wherein said step of producing a probability table further comprises determining a uniformly distributed random number for each of said one or more diagnosis-prescription combinations.
Embodiment 13 constitutes a method , in particular of embodiment 7, wherein said step (b) further comprises updating said probability table.
Embodiment 14 constitutes a method , in particular of embodiment 13, wherein said updating step comprises: (a) receiving at least one current diagnosis-prescription combination; (b) determining a current frequency of occurrence for said at least one current diagnosis-prescription combination; and (c) updating said probability table using said determined current frequency of occurrence.
Embodiment 15 constitutes a method, in particular of embodiment 14, wherein said updating step further comprises combining said at least one current frequency of occurrence with existing frequencies of occurrence, and determining an updated diagnostic rank of occurrence for each of said one or more diagnosis-prescription combinations.
Embodiment 16 constitutes a method , in particular of embodiment 15, wherein said step of updating a probability table further comprises determining an updated prescription rank of occurrence for each of said one or more diagnosis-prescription combinations.
Embodiment 17 constitutes a method , in particular of embodiment 16, wherein said step of updating a probability table further comprises determining an updated uniformly distributed random number for each of said one or more diagnosis-prescription combinations.
Embodiment 18 constitutes a method, in particular of embodiment 1, wherein said step (c) further comprises separating, from said one or more diagnosis-prescription combinations, one or more diagnosis-prescription combinations having one-to-one or one-to-many relationships.
Embodiment 19 constitutes a method , in particular of embodiment 18, wherein said step (c) further comprises applying a linking algorithm to link a group of one or more diagnostic information records with a group of one or more prescription information records.
Embodiment 20 constitutes a method, in particular of embodiment 18, wherein said step (c) comprises applying a maximum likelihood algorithm to link a group of one or more of diagnostic information records with a group of one or more prescription information records.
Embodiment 21 constitutes a method, in particular of embodiment 18, wherein said step (c) comprises applying a relative likelihood algorithm to link a group of one or more diagnostic information records with a group of one or more of prescription information records.
Embodiment 22 constitutes a method , in particular of embodiment 18, wherein said step (c) comprises applying a second loop algorithm to link a group of one or more diagnostic information records with a group of one or more prescription information records.
Embodiment 23 constitutes a method, in particular of embodiment 18, wherein said step (c) comprises applying manual linking to link a group of one or more diagnostic information records with a group of one or more prescription information records.
Embodiment 24 constitutes a method , in particular of embodiment 1, further comprising the step of retrieving said plurality of diagnostic information records and said plurality of prescription information records before step (a) from one or more sample providers.
Embodiment 25 constitutes a system for creating data links between a plurality of diagnostic information records and a plurality of prescription information records, comprising: a) means for analyzing said plurality of diagnostic information records and said plurality of prescription information records to derive one or more diagnosis-to-prescription relationships, each relating a group of one or more of said diagnostic information records to a group of one or more, if any, of said prescription information records; b) means, coupled to said analyzing means and receiving said derived diagnosis-to-prescription relationships therefrom, for determining one or more correspondence probabilities, each using at least one of said one or more received diagnosis-to-prescription relationships and indicating a correspondence between a group of one or more of said diagnostic information records and a group of one or more of said prescription information records; and c) means, coupled to said determining means and receiving said determined correspondence probabilities, for linking one or more of said diagnostic information records to one or more of said prescription information records using said one or more correspondence probabilities determined by said determining means.
Embodiment 26 constitutes a system, in particular of embodiment 25, further comprising means, coupled to said deriving means, for providing one or more historical relationships, each signifying an existing relationship between a group of one or more of said diagnostic information records and a group of one or more of said prescription information records, and wherein said means for determining one or more correspondence probabilities comprises means for determining a correspondence probability between a group of one or more of said diagnostic information records and a group of one or more of said prescription information records using one or more of said relationships derived by said deriving means and said one or more historical relationship records.
Embodiment 27 constitutes a system , in particular of embodiment 26, wherein said one or more diagnosis-to-prescription relationships comprise one-to-one relationships.
Embodiment 28 constitutes a system, in particular of embodiment 27, wherein said one or more diagnosis-to-prescription relationships comprise one-to-many relationships.
Embodiment 29 constitutes a system , in particular of embodiment 28, wherein said one or more diagnosis-to-prescription relationships comprise many-to-one relationships.
Embodiment 30 constitutes a system , in particular of embodiment 29, wherein said one or more diagnosis-to-prescription relationships comprise many-to-many relationships.
Embodiment 31 constitutes a system , in particular of embodiment 25, wherein said means for determining one or more correspondence probabilities further comprises means, coupled to said analyzing means and receiving one or more of said diagnostic information records and one or more of said prescription information records, for producing a probability table using said one or more correspondence probabilities.
Embodiment 32 constitutes a system , in particular of embodiment 31, wherein said means for producing a probability table further comprises means for determining one or more diagnosis-prescription combinations using said plurality of diagnostic information records and said plurality of prescription information records.
Embodiment 33 constitutes a system , in particular of embodiment 32, wherein said means for producing a probability table further comprises means, coupled to said determining means, for calculating a frequency of occurrence for each diagnosis-prescription combination.
Embodiment 34 constitutes a system , in particular of embodiment 33, wherein said means for producing a probability table further comprises means, coupled to said calculating means and receiving frequencies of occurrence therefrom, for determining a diagnostic rank of occurrence for each diagnosis-prescription combination.
Embodiment 35 constitutes a system, in particular of embodiment 34, wherein said means for producing a probability table further comprises means, coupled to said calculating means and receiving frequencies of occurrence therefrom, for determining a prescription rank of occurrence for each diagnosis-prescription combination.
Embodiment 36 constitutes a system , in particular of embodiment 35, wherein said means for producing a probability table further comprises means, coupled to said calculating means and receiving frequencies of occurrence therefrom, for determining a uniformly distributed random number for each diagnosis-prescription combination.
Embodiment 37 constitutes a system , in particular of embodiment 36, wherein said means for determining a correspondence probability further comprises means, coupled to said deriving means, for updating said probability table.
Embodiment 38 constitutes a system , in particular of embodiment 32 wherein said means for updating said probability table further comprises: (a) means, coupled to said determining means, for calculating a current frequency of occurrence for said at least one current diagnosis-prescription combination; and (c) means, coupled to said calculating means, for updating said probability table using said determined current frequency of occurrence.
Embodiment 39 constitutes a system , in particular of embodiment 38, wherein said means for updating a probability table further comprises means, coupled to said determining means, for combining said at least one current frequency of occurrence with said determined frequencies of occurrence, and determining an updated diagnostic rank of occurrence for each diagnosis-prescription combination.
Embodiment 40 constitutes a system , in particular of embodiment 39, wherein said means for updating a probability table further comprises means, coupled to said determining means, for determining an updated prescription rank of occurrence for each diagnosis-prescription combination.
Embodiment 41 constitutes a system , in particular of embodiment 39, wherein said means for updating a probability table further comprises means, coupled to said determining means, for determining a uniformly distributed random number for each diagnosis-prescription combination.
Embodiment 42 constitutes a system , in particular of embodiment 25, wherein said linking means further comprises means, coupled to said determining means and receiving said plurality of diagnostic information records and said plurality of prescription information records, for separating each of said plurality of diagnostic information records and said plurality of prescription information records having either of one-to-one and one-to-many relationships,
Embodiment 43 constitutes a system , in particular of embodiment 42, wherein said linking means further comprises means, coupled to said determining means and receiving correspondence probabilities therefrom, for applying a linking algorithm to link said plurality of diagnostic information records and said plurality of prescription information records.
Embodiment 44 constitutes a system , in particular of embodiment 25, further comprising means, coupled to said analyzing means, for retrieving said plurality of diagnostic information records and said plurality of prescription information records from one or more sample providers and providing said analyzing means therewith.

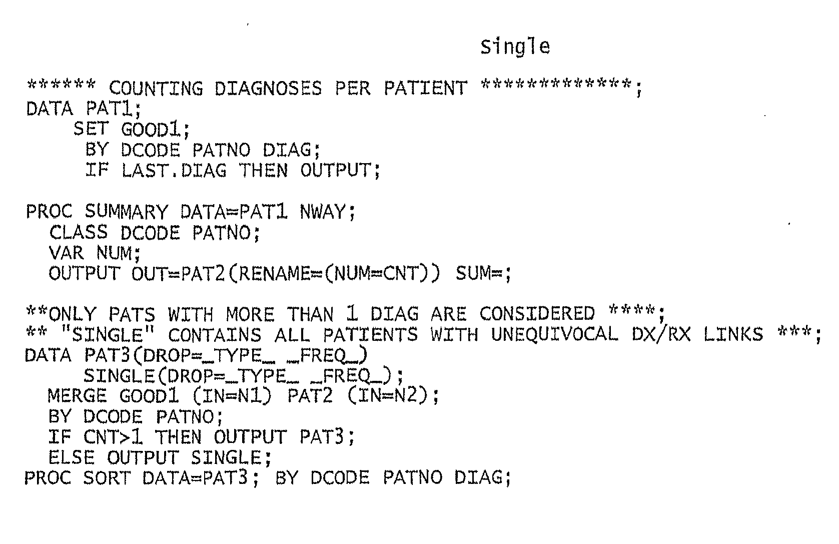

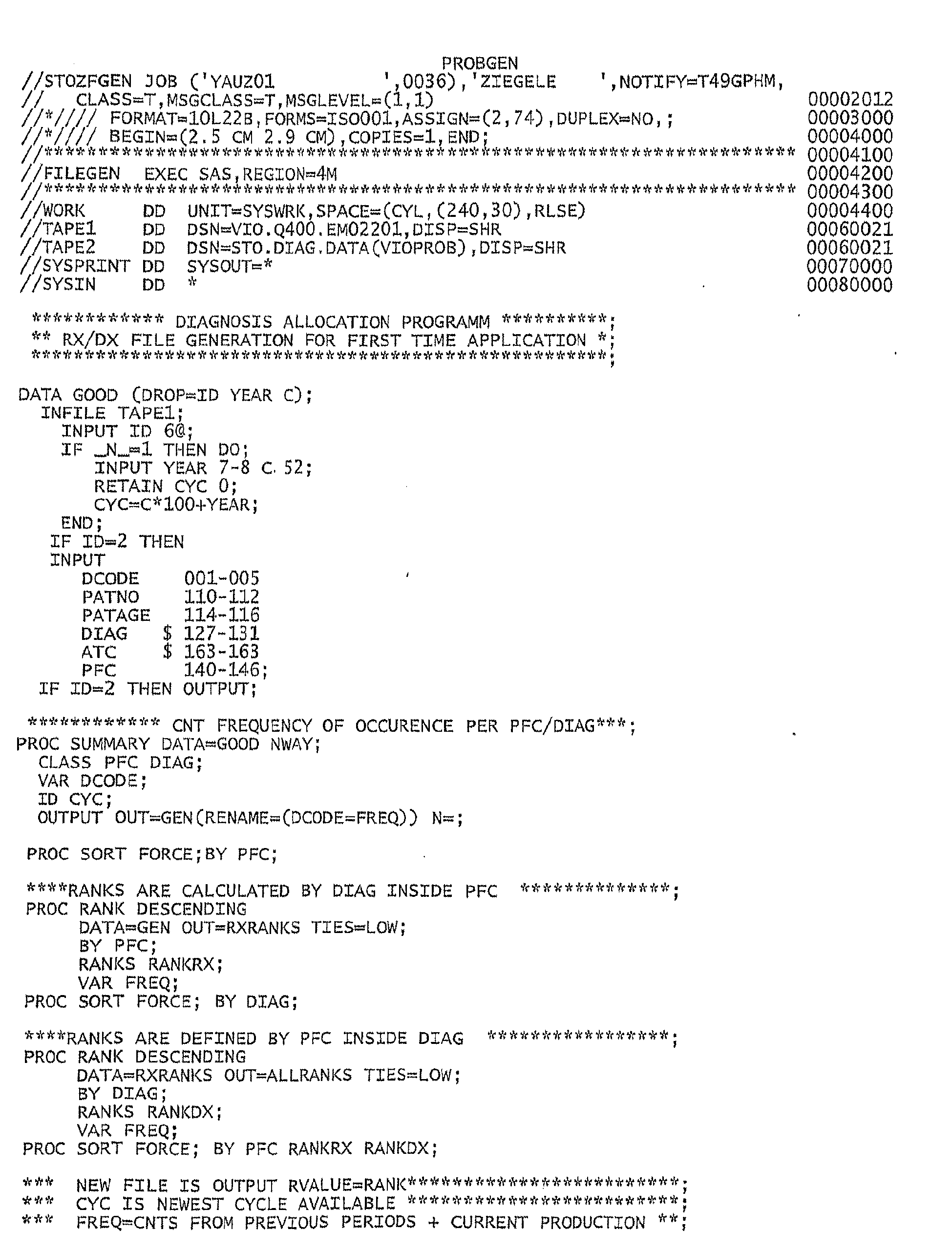

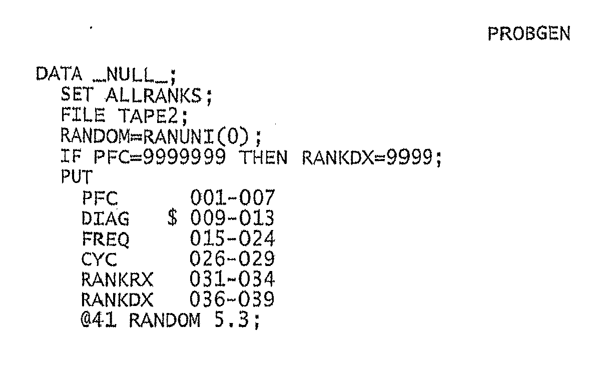

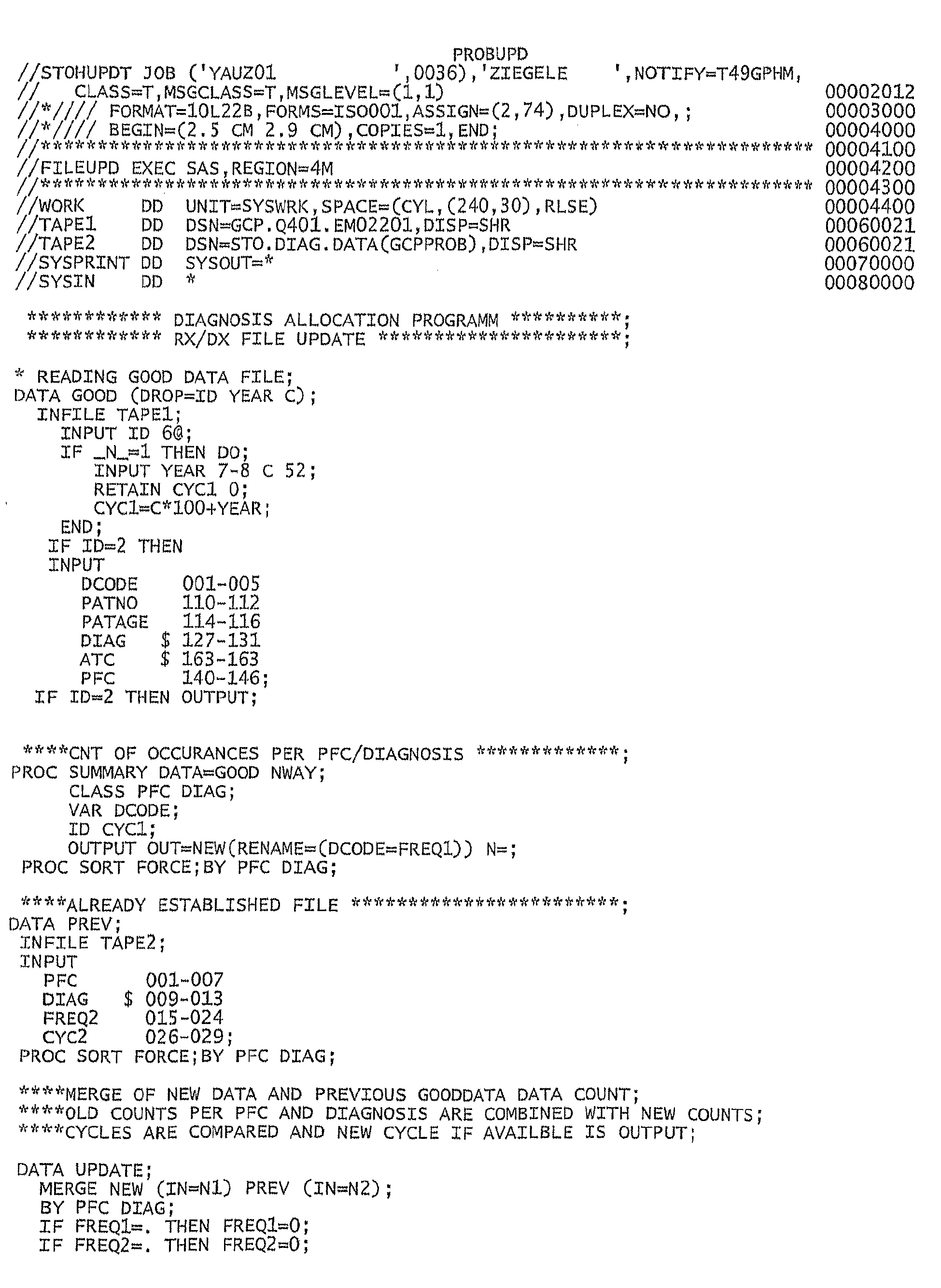

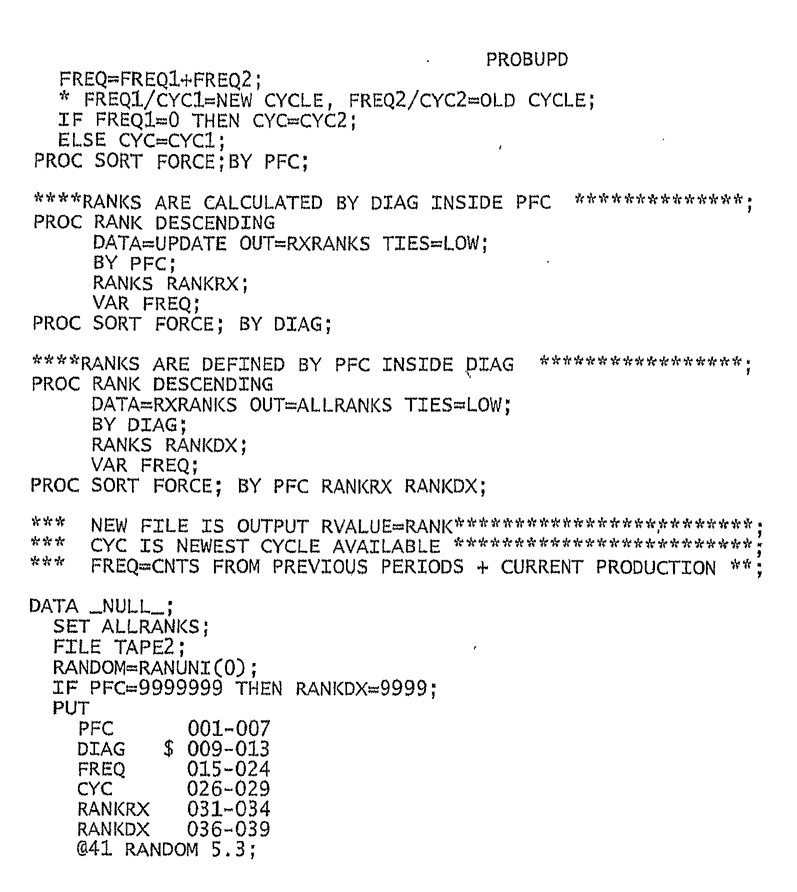

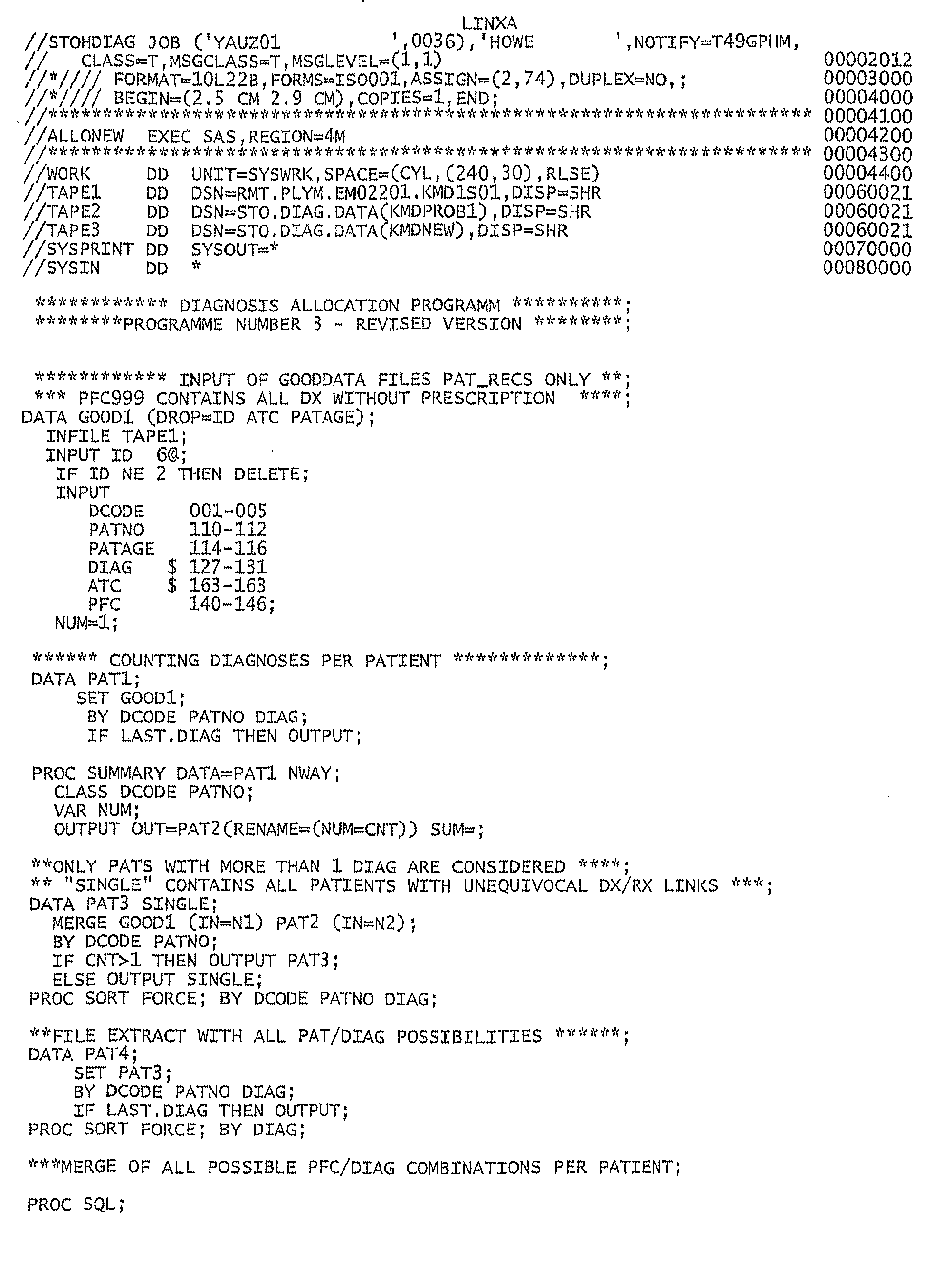

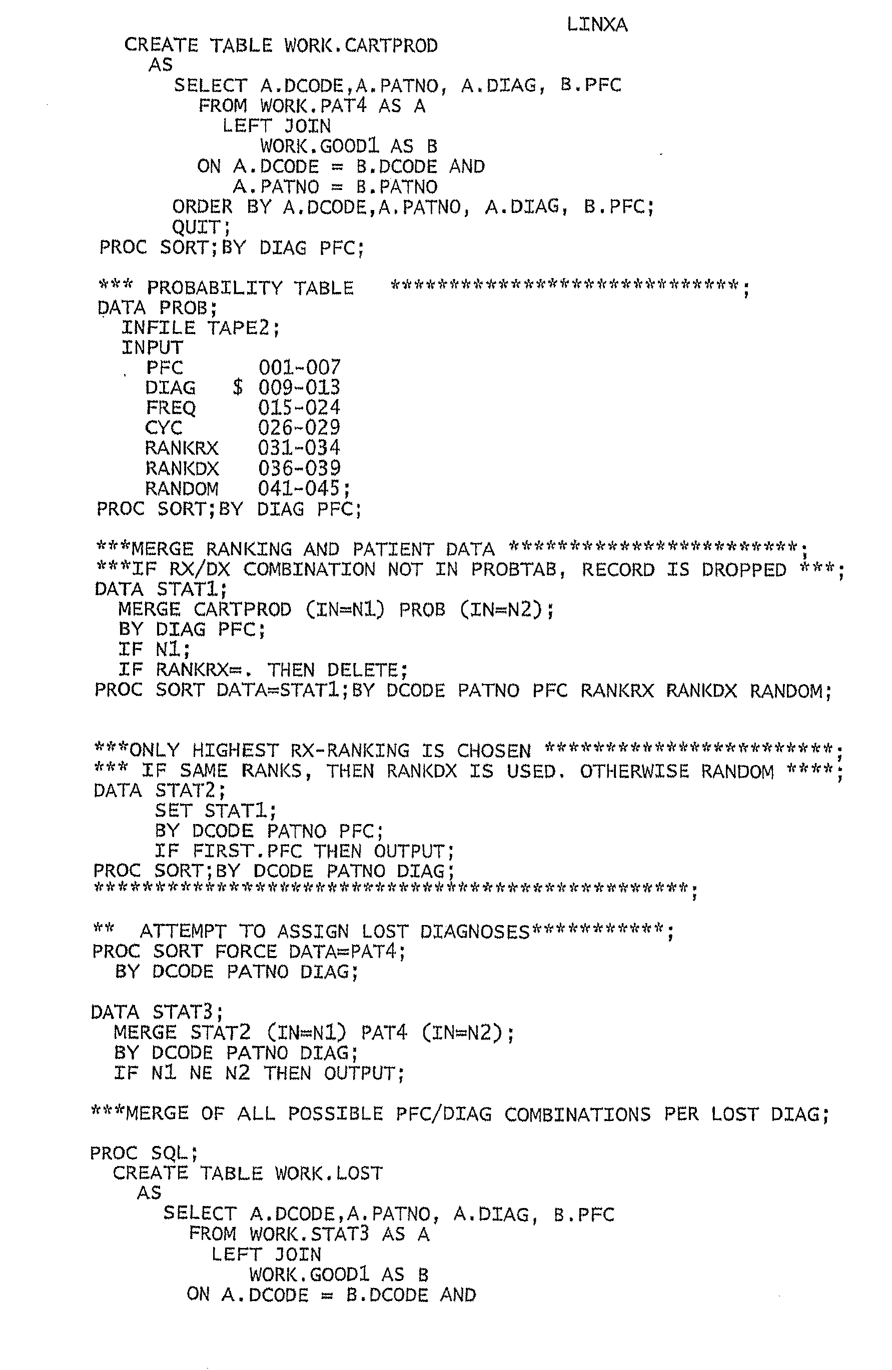

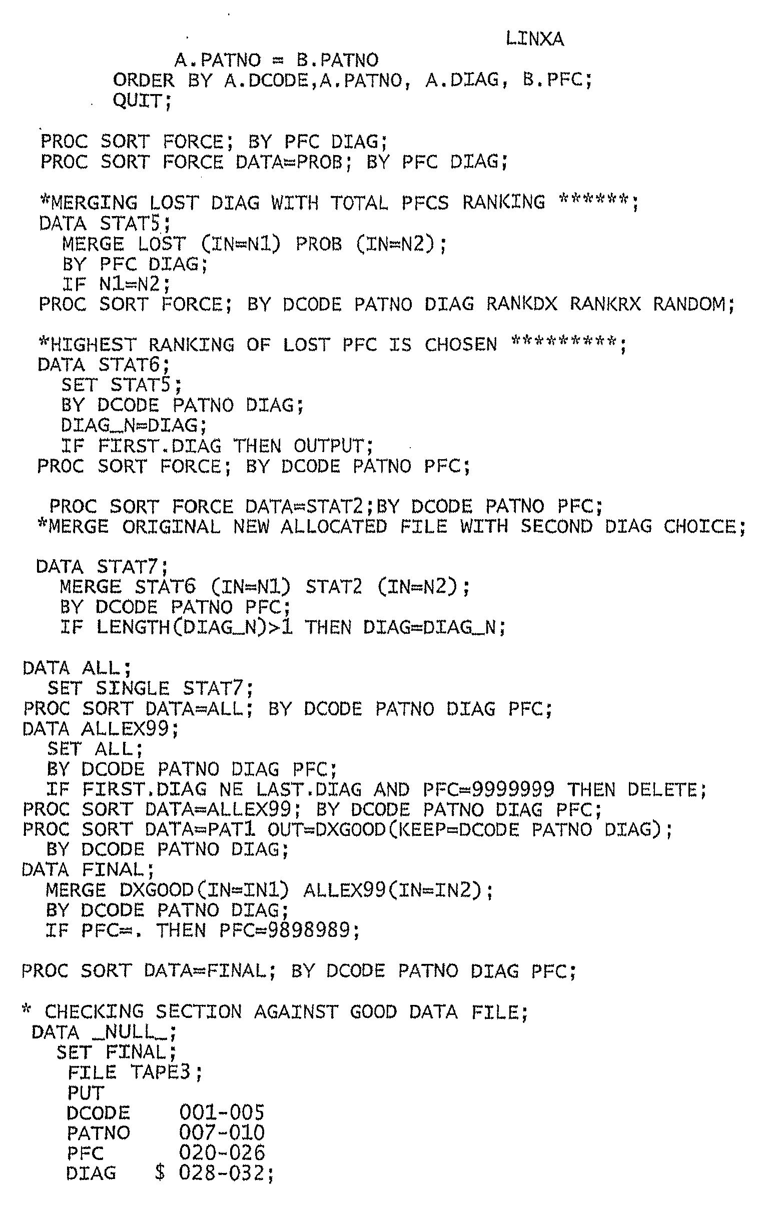

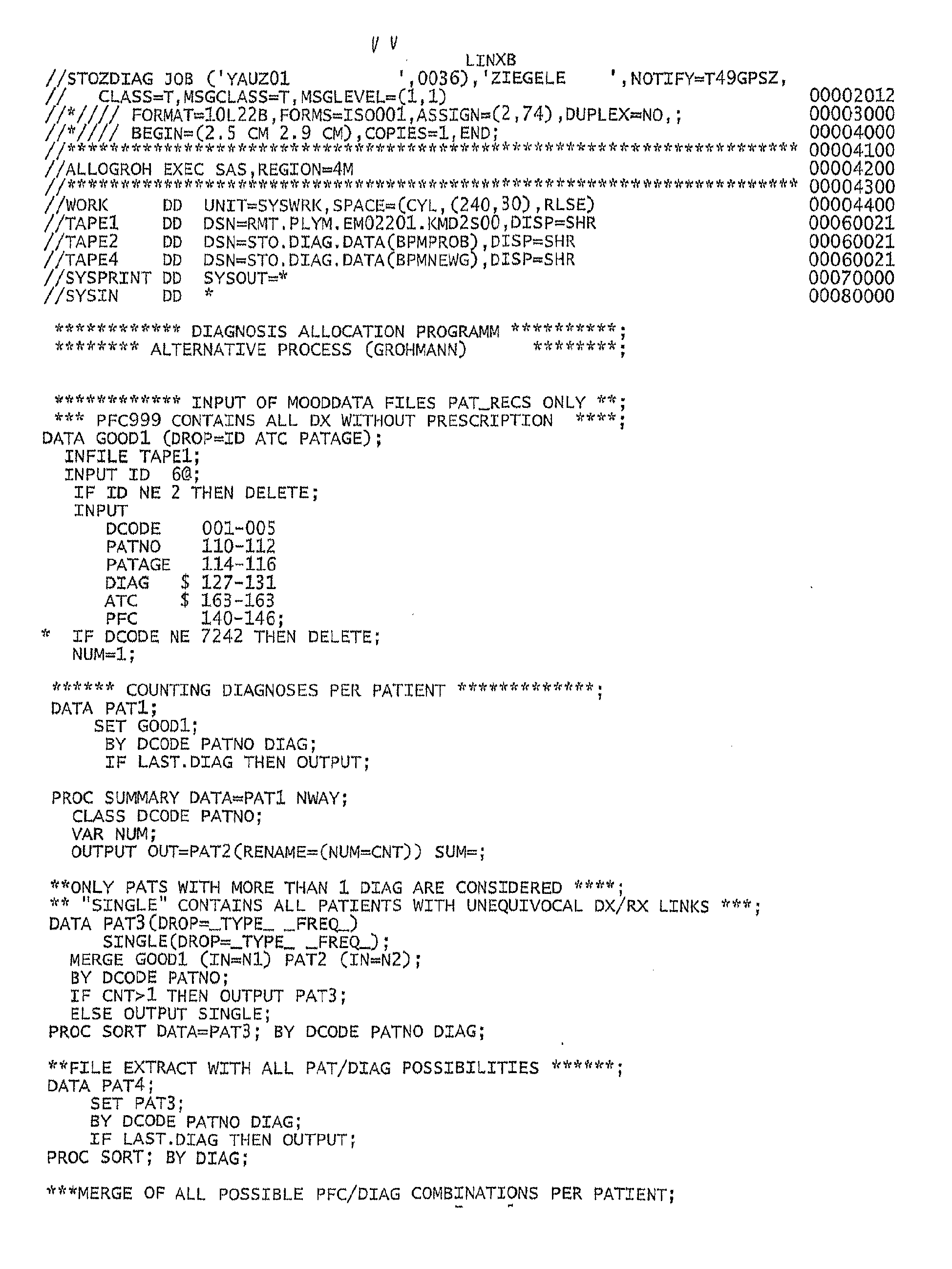

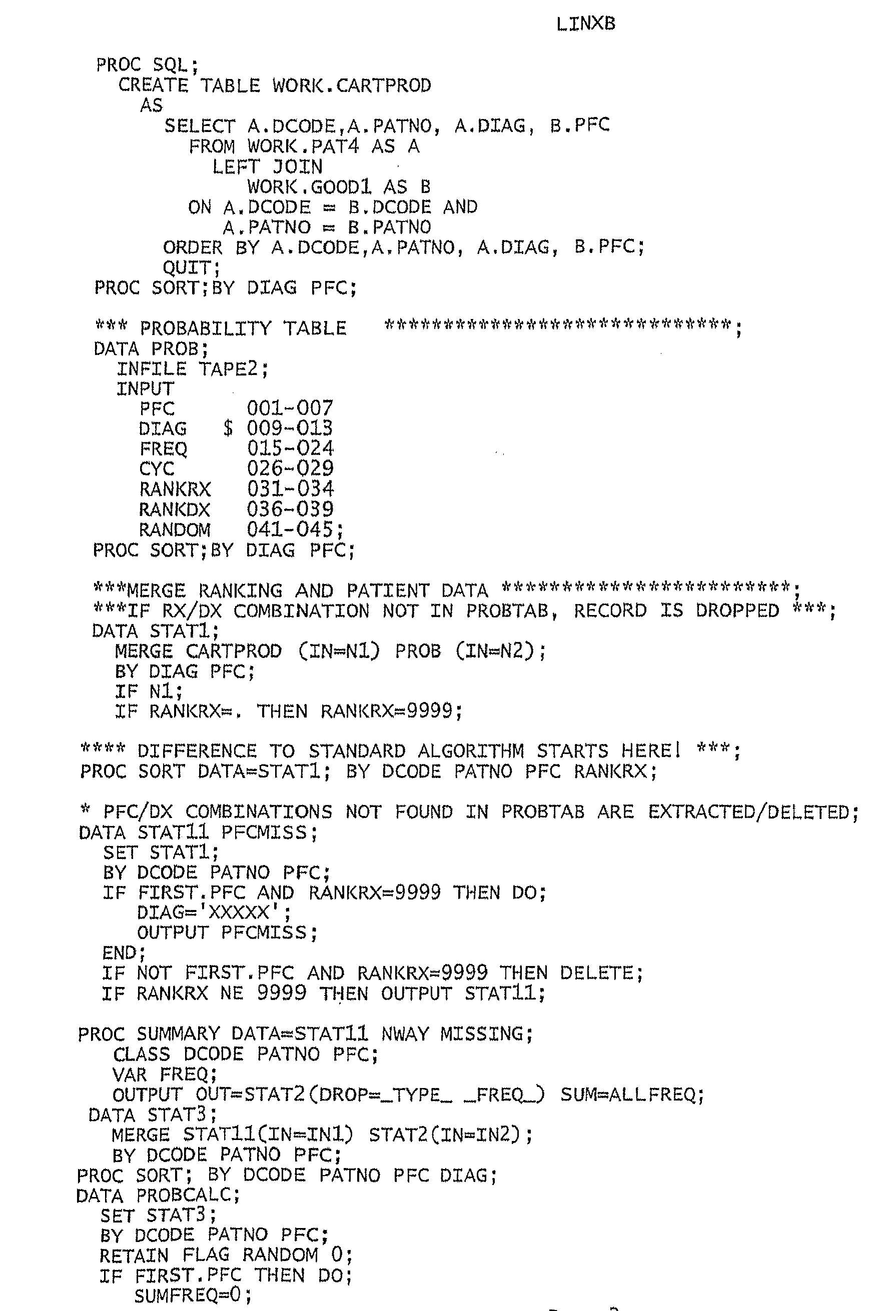

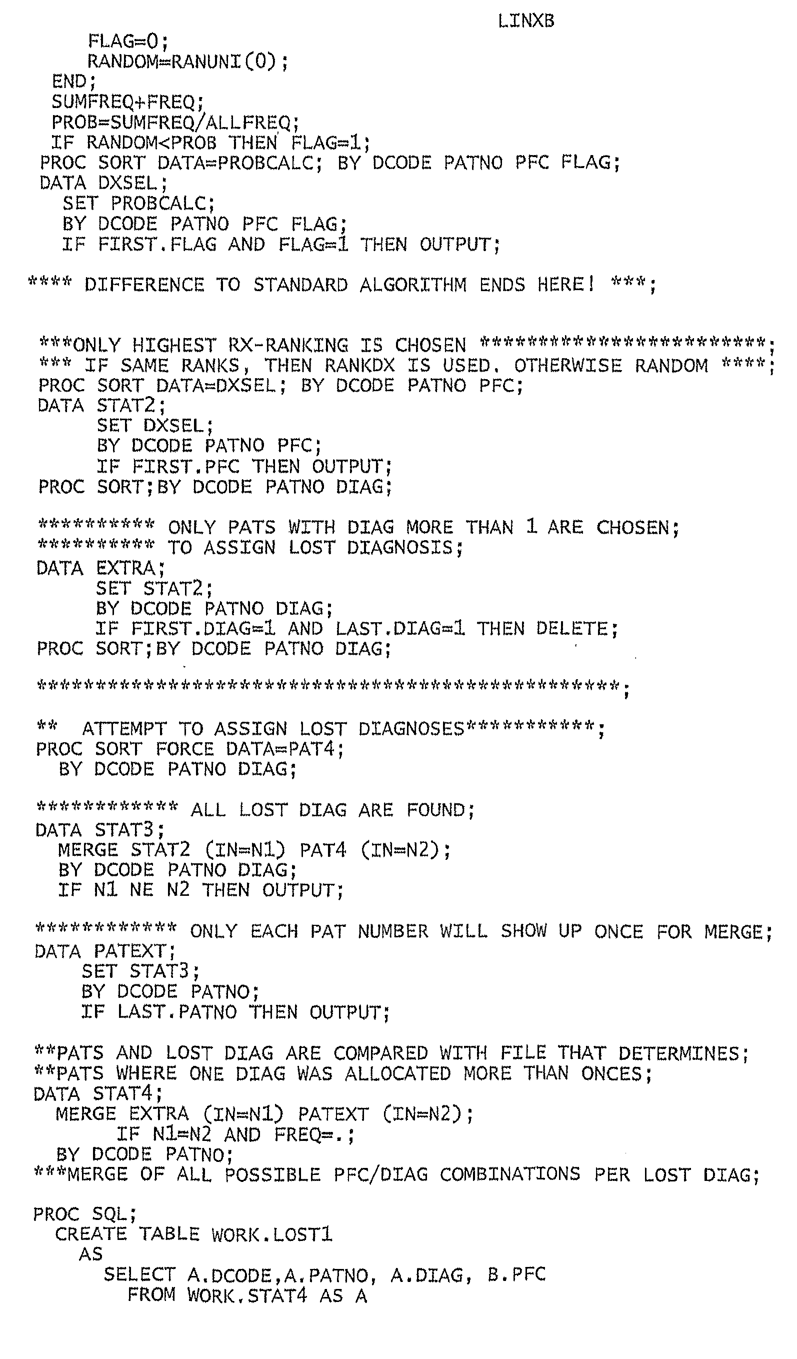

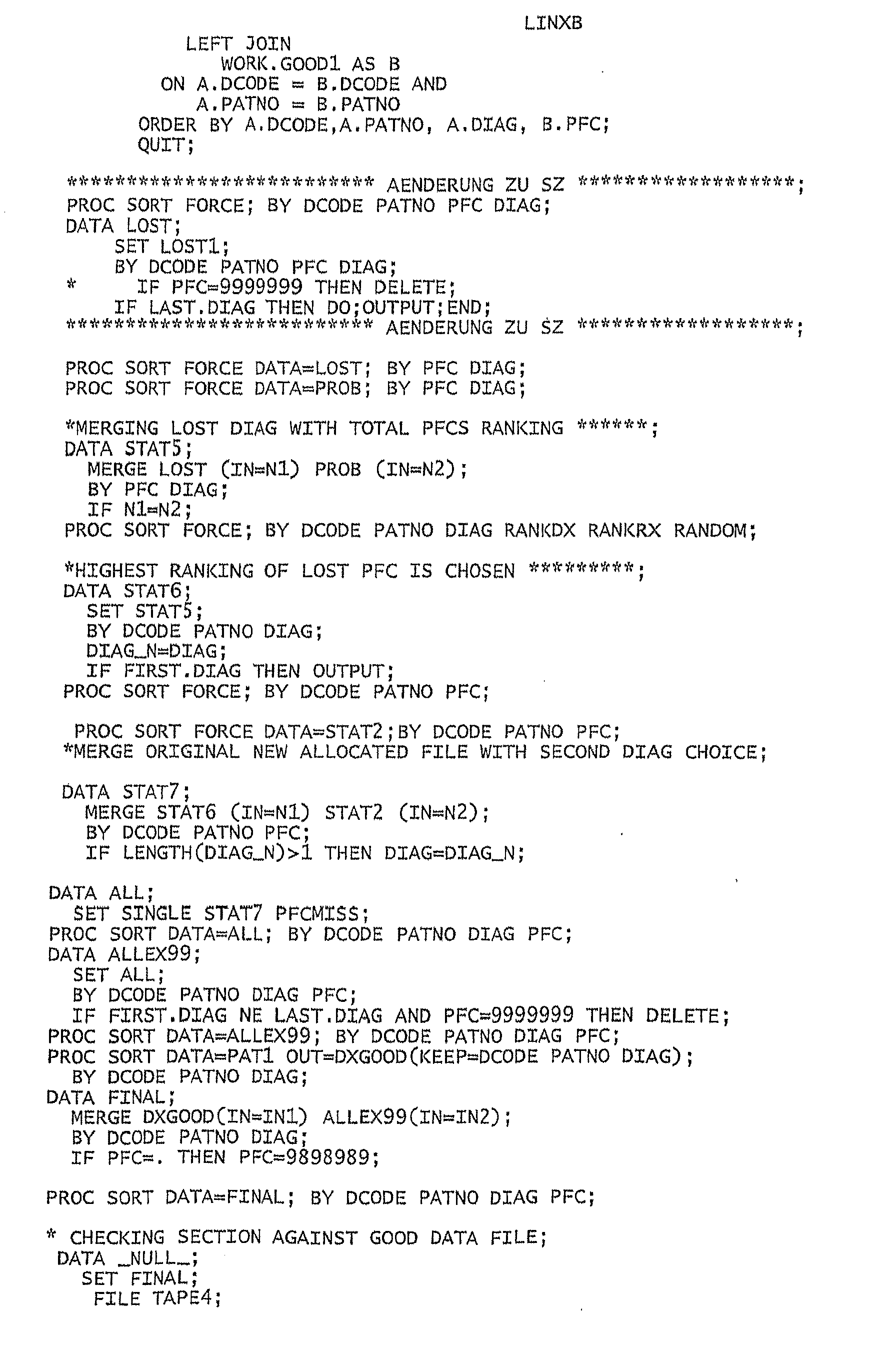

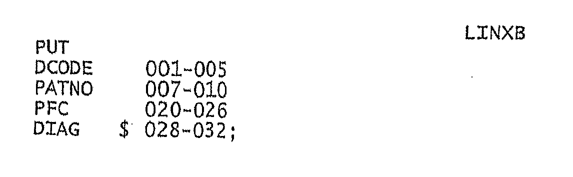

## Claims

1. A method for creating data links in a computer database between a plurality of diagnostic information records and a plurality of prescription information records, comprising the steps of:
a) analyzing said plurality of diagnostic information records and said plurality of prescription information records, to derive one or more diagnosis-to-prescription relationships, each relating a group of one or more of diagnostic information records to a group of one or more, if any, of prescription information records;
b) calculating a correspondence probability for each relationship derived in step (a) and indicating a correspondence between a group of one or more of said diagnostic information records and a group of one or more of said prescription information records, wherein calculating the correspondence probabilities comprises:
b1) selecting a sub-set of current diagnostic and prescription information records containing only those diagnostic and prescription information records which are related to each other by a one-to-one and/or one-to-many relationship as derived in step a);
b2) determining a frequency of occurrence for the occurrence of each diagnosis-to-prescription relationship;
b3) determining a diagnostic rank of occurrence of a particular diagnostic information record from a list of all diagnostic information records corresponding to a particular prescription information record;
b4) determining a prescription rank of occurrence of a particular prescription information record from a list of all prescription information records corresponding to particular diagnostic information record in updated historical data;
b5) determining the one or more correspondence probabilities for each relationship derived in step (a) based on the frequency of occurrence and the ranks determined in steps b2 - b4, respectively;
c) producing a probability table comprising the correspondence probability information calculated in step b);
d) separating out all combinations of diagnostic and prescription information records having one-to-one and/or one-to-many relationships;
e) linking one or more of said remaining diagnostic information records having many-to-one and/or many-to-many relationships with said prescription information records to one or more of said prescription information records using said one or more correspondence probabilities from the probability table.

2. The method of Claim 1, further comprising the step of providing one or more historical relationships prior to step (a), each signifying a relationship between a group of one or more of said diagnostic information records and a group of one or more of said prescription information records, and wherein said step (b) further comprises determining one or more correspondence probabilities, each using one or more of said diagnosis-to-prescription relationships derived in step (a) and said one or more historical relationships.

3. The method of Claim 1, wherein said step of producing a probability table comprises determining one or more diagnosis-prescription combinations using said plurality of diagnostic information records and said plurality of prescription information records.

4. The method of Claim 3, wherein said step of producing a probability table further comprises calculating a frequency of occurrence for each of said one or more diagnosis-prescription combinations.

5. The method of Claim 4, wherein said step of producing a probability table further comprises using said frequency of occurrence to determine a diagnostic rank of occurrence for each of said one or more diagnosis-prescription combinations.

6. The method of Claim 5, wherein said step of producing a probability table further comprises using said frequency of occurrence to determine a prescription rank of occurrence for each of said one or more diagnosis-prescription combinations.

7. The method of Claim 6, wherein said step of producing a probability table further comprises determining a uniformly distributed random number for each of said one or more diagnosis-prescription combinations.

8. The method of Claim 1, wherein said step (b) further comprises updating said probability table.

9. The method of Claim 8, wherein said updating step comprises:
(a) receiving at least one current diagnosis-prescription combination;
(b) determining a current frequency of occurrence for said at least one current diagnosis-prescription combination; and
(c) updating said probability table using said determined current frequency of occurrence.

10. The method of Claim 9, wherein said updating step further comprises combining said at least one current frequency of occurrence with existing frequencies of occurrence, and determining an updated diagnostic rank of occurrence for each of said one or more diagnosis-prescription combinations.

11. The method of Claim 10, wherein said step of updating a probability table further comprises determining an updated prescription rank of occurrence for each of said one or more diagnosis-prescription combinations.

12. The method of Claim 11, wherein said step of updating a probability table further comprises determining an updated uniformly distributed random number for each of said one or more diagnosis-prescription combinations.

13. The method of Claim 1, wherein said step (e) further comprises applying a linking algorithm to link a group of one or more diagnostic information records with a group of one or more prescription information records,wherein the linking algorithm is a maximum-likelihood algorithm, or wherein the linking algorithm is a relative-likelihood algorithm.

14. The method of Claim 13, wherein said step (e) further comprises applying a second loop algorithm to link a group of one or more diagnostic information records with a group of one or more prescription information records, and/orwherein said step (e) further comprises applying manual linking to link a group of one or more diagnostic information records with a group of one or more prescription information records.

15. A system for creating a computer database of diagnostic and prescription information records having data links between a plurality of diagnostic information records and a plurality of prescription information records, comprising:
a) means for analyzing said plurality of diagnostic information records and said plurality of prescription information records,,̅ to derive one or more diagnosis-to-prescription relationships, each relating a group of one or more of diagnostic information records to a group of one or more, if any, of prescription information records;
b) means, coupled to said analyzing means and receiving said derived diagnosis-to-prescription relationship information therefrom, for determining a correspondence probability for each relationship derived in step (a) and indicating a correspondence between a group of one or more of said diagnostic information records and a group of one or more of said prescription information records, wherein determining the correspondence probabilities comprises:
b1) selecting a sub-set of current diagnostic and prescription information records containing only those diagnostic and prescription information records which are related to each other by a one-to-one and/or one-to-many relationship as derived in step a);
b2) determining a frequency of occurrence for the occurrence of each diagnosis-to-prescription relationship;
b3) determining a diagnostic rank of occurrence of a particular diagnostic information record from a list of all diagnostic information records corresponding to a particular prescription information record;
b4) determining a prescription rank of occurrence of a particular prescription information record from a list of all prescription information records corresponding to particular diagnostic information record in updated historical data;
b5) determining the one or more correspondence probabilities for each relationship derived in step (a) based on the frequency of occurrence and the ranks determined in steps b2 - b4, respectively;
c1) means, coupled to said determining means for separating out all combinations of diagnostic and prescription information records having one-to-one and/or one-to-many relationships;
c) further means, coupled to said determining means and receiving said determined correspondence probabilities, for linking one or more of said diagnostic information records to one or more of said prescription information records using said one or more correspondence probabilities determined by said determining means.
